# EUROPÄISCHE PATENTANMELDUNG

(11) **EP 0 798 560 A2**
(43) Veröffentlichungstag der Anmeldung: **01.10.1997**
(21) Anmeldenummer: 97105158.6
(22) Anmeldetag: 26.03.1997
(51) Int. Cl.: G01N 33/00, G01N 27/12

(54) **Messvorrichtung sowie Verfahren zu deren Betrieb**

(30) Priorität: 29.03.1996 DE 19612706
(71) Anmelder: Sick AG, 79183 Waldkirch, Breisgau (DE)
(72) Erfinder: Herrmann, Volker, Dr., 01665 Klipphausen (DE); Eschrich, Robert, Dr., 01796 Pirna (DE)
(74) Vertreter: Finsterwald, Manfred, Dipl.-Ing., Dipl.-Wirtsch.-Ing.

(57) **Zusammenfassung**

Die Erfindung betrifft eine Meßvorrichtung zur Messung der Konzentration eines bestimmten Stoffes in einem von einem Meßfluid durchströmten Meßvolumen, insbesondere der Kohlenmonoxid-Konzentration in einem heißen, staubbeladenen Abgas. Die Meßvorrichtung umfaßt eine Stoffsensoranordnung mit wenigstens einem schnell ansprechenden Innensensor 1, 2 mit Kurzzeitstabilität innerhalb des Meßvolumens 5 und wenigstens einem langsam ansprechenden Außensensor 3 hoher Meßgenauigkeit außerhalb des Meßvolumens 5 sowie eine mit den Sensoren 1, 2, 3 verbundene Schaltungs- und Auswerteeinheit 4, welche die Einzelsignale der Sensoren 1, 2, 3 empfängt und aus den empfangenen Einzelsignalen einen den momentanen Wert der Stoffkonzentration im Meßfluid mit hoher Genauigkeit repräsentierenden Meßwert ableitet. Die Erfindung betrifft außerdem ein Verfahren zum Betrieb einer derartigen Meßvorrichtung, bei dem die Schaltungs- und Auswerteeinheit 4 das Einzelsignal des Innensensors 1, 2 mithilfe des genaueren Außensensorsignals kalibriert und das kalibrierte Innensensorsignal der Meßwertermittlung zugrunde legt.

## Beschreibung

Die Erfindung betrifft eine Meßvorrichtung zur Messung der Konzentration eines bestimmten Stoffes in einem von einem Meßfluid durchströmten Meßvolumen, insbesondere der Kohlenmonoxid-Konzentration in einem heißen, staubbeladenen Abgas. Die Erfindung betrifft außerdem ein Verfahren zum Betrieb einer derartigen Meßvorrichtung.

Meßvorrichtungen dieser Art werden beispielsweise dafür verwendet, die Konzentration von Kohlenmonoxid als nachzuweisender Suchstoff in den Abgasen einer Verbrennungs- oder Filteranlage zu bestimmen, um den das Kohlenmonoxid erzeugenden Prozeß, die gesamte Anlage oder das Filtersystem der Anlage beim Überschreiten einer bestimmten Kohlenmonoxid-Konzentration abschalten zu können.

Problematisch sind dabei die mitunter auftretenden, schnell ansteigenden Stoffkonzentrationsspitzen, für deren Nachweis schnell ansprechende Sensoren erforderlich sind. Viele dieser Konzentrationsspitzen sind unkritisch. Einige können jedoch einen Grenzwert überschreiten, ab welchem Explosionsgefahr besteht.

Wenn ein langsam ansprechender Sensor dem tatsächlichen Wert der Stoffkonzentration nicht schnell genug folgen kann, wird aus Sicherheitsgründen bereits bei einem Bruchteil der kritischen Stoffkonzentration abgeschaltet. Der Grund dafür ist, daß zum Zeitpunkt einer durch den langsam ansprechenden Sensor angezeigten Meßwerterhöhung nicht feststellbar ist, wie weit die Stoffkonzentration tatsächlich bereits angestiegen ist. Die Folge davon ist, daß in der Praxis der den nachzuweisenden Stoff erzeugende Prozeß, die gesamte Anlage oder deren Filtersystem unnötig oft abgeschaltet wird, wodurch hohe Kosten verursacht werden.

Es sind derzeit keine Sensoren bekannt, die sowohl eine genügend kurze Ansprechzeit als auch eine ausreichend hohe Meßgenauigkeit aufweisen.

Mit Ansprechzeit ist hier grundsätzlich diejenige für einen erfindungsgemäß verwendbaren Sensor charakteristische Zeitspanne gemeint, innerhalb welcher das Ausgangssignal des Sensors eine 90% der tatsächlichen Stoffkonzentration entsprechende Signalhöhe erreicht hat. Diese Ansprechzeit wird im folgenden mit t₉₀ abgekürzt.

Eine Aufgabe der Erfindung ist es, eine Meßvorrichtung sowie ein Verfahren für deren Betrieb zu schaffen, wodurch die Messung des zeitlichen Verhaltens des Absolutwertes der Konzentration eines bestimmten Stoffes in einem Meßfluid mit möglichst hoher Genauigkeit auch bei hohen Meßfluid-Temperaturen und bei stark mit insbesondere partikelförmigen Verunreinigungen belasteten Meßfluiden ermöglicht wird.

Gelöst wird diese Aufgabe erfindungsgemäß im wesentlichen durch eine Stoffsensoranordnung mit wenigstens einem schnell ansprechenden Innensensor mit Kurzzeitstabilität innerhalb des Meßvolumens und wenigstens einem langsam ansprechenden Außensensor hoher Meßgenauigkeit außerhalb des Meßvolumens, und eine mit den Sensoren verbundene Schaltungs- und Auswerteeinheit, welche die Einzelsignale der Sensoren empfängt und aus den empfangenen Einzelsignalen einen den momentanen Wert der Stoffkonzentration im Meßfluid mit hoher Genauigkeit repräsentierenden Meßwert ableitet.

Erfindungsgemäß werden demnach mehrere Sensoren mit unterschiedlichen Eigenschaften in einander ergänzender Weise eingesetzt, um gemeinsam mittels einer geeignet ausgebildeten Schaltungs- und Auswerteeinheit das zeitliche Verhalten der Stoffkonzentration im Meßfluid mit hohen Genauigkeit zu ermitteln.

Mit Kurzzeitstabilität ist der Umstand gemeint, daß die vom Innensensor gelieferten Ausgangssignale von langsam veränderlichen Größen wie beispielsweise Temperatur und Strömungsgeschwindigkeit des Meßfluids beeinflußbar sind. Die vom Innensensor angezeigten Absolutwerte der Stoffkonzentration im Meßfluid sind daher nur über einen vergleichsweise kurzen Zeitraum stabil.

Der Außensensor dagegen benötigt mehr Zeit für jede Messung und ist schon aus diesem Grund relativ unempfindlich für sich langsam verändernde Einflußgrößen.

Gemäß einer Ausführungsform der Erfindung ist der außerhalb des Meßvolumens befindliche Außensensor über einen extraktiven Meßkanal mit dem Meßvolumen verbunden. Über diesen Meßkanal ist dem Meßvolumen ein Meßfluidteilstrom entnehmbar und dem Außensensor zur genauen Messung der Stoffkonzentration zuführbar. Der Meßkanal umfaßt vorzugsweise ein Entnahmerohr, dessen im Meßvolumen gelegenes Ende mit einem Ansaugfilter versehen ist, eine Gasfördereinrichtung, einen Gaskühler und einen Kondensatabscheider.

Auf diese Weise können außerhalb des Meßvolumens solche Stoffsensoren mit einer sehr hohen Meßgenauigkeit eingesetzt werden, bei denen das Meßfluid eine bestimmte Temperatur nicht überschreiten darf und im wesentlichen frei von partikelförmigen Verunreinigungen und Kondensaten sein muß.

Der Kondensatabscheider weist bevorzugt zwei Auslässe auf, wobei von einem Meßauslaß eine Bypassleitung zum Außensensor und von dort weiter zur Gasfördereinrichtung führt und ein Kondensatauslaß direkt mit der Gasfördereinrichtung durch eine Abführleitung verbunden ist. Über die Bypassleitung wird trockenes, von dem Kondensat im wesentlichen befreites Meßfluid dem Außensensor zur Messung zugeführt, während der Abtransport des aus dem Meßfluid entfernten Kondensats über die Abführleitung erfolgt.

Gemäß einer weiteren bevorzugten Weiterbildung der Erfindung ist der Meßkanal mit einer Luftzufuhranordnung versehen. Diese gestattet eine vorzugsweise periodische Beaufschlagung des Außensensors mit Umgebungsluft. Auf diese Weise ist es möglich, für Erholungsphasen für den Außensensor so sorgen. Des weiteren gestattet die Umgebungsluftzufuhr die Durchführung von Nullpunktprüfungen des Außensensors.

Für den Fall, daß diejenige Temperatur, der bestimmte Teile des innerhalb des Meßvolumens angeordneten Innensensors maximal ausgesetzt werden dürfen, niedriger als die erwartete Temperatur des Meßfluids ist, kann gemäß einer Weiterbildung der Erfindung eine Tauchsonde für den Innensensor vorgesehen sein, in der die temperaturempfindlichen Teile des Innensensors vor dem Meßfluid geschützt untergebracht sind. Dabei ist der für den nachzuweisenden Stoff empfindliche Bereich des Innensensors bevorzugt außerhalb der Tauchsonde derart angeordnet, daß er direkt vom Meßfluid umspült werden kann.

Gemäß einer weiteren bevorzugten Ausführungsform der Erfindung ist für den Sensor eine mit einer Druckluftleitung gekoppelte Reinigungsdüse vorgesehen, die mittels gerichteter Druckluftstöße eine vorzugsweise zyklische Abreinigung des sensitiven Bereiches des Innensensors ermöglicht. Diese Reinigungseinrichtung ermöglicht es, einer abnehmenden Eignung des Innensensors für die Messung der Stoffkonzentration entgegenzuwirken, indem der sensitive Bereich von durch Staub oder anderen Verunreinigungen gebildeten Ablagerungen gezielt befreit wird.

Von besonderem Vorteil ist diese Möglichkeit der Innensensor-Reinigung dann, wenn gemäß einer bevorzugten Weiterbildung der erfindungsgemäßen Meßvorrichtung mehrere Innensensoren innerhalb des Meßvolumens verwendet werden, da dann während der Phasen, in denen einer oder mehrere Innensensoren abgereinigt werden, wenigstens ein weiterer Innensensor damit fortfahren kann, Meßwerte an die Schaltungs- und Auswerteeinheit zu liefern. Auf diese Weise wird eine ununterbrochene Messung der Stoffkonzentration im Meßfluid ermöglicht.

Vorzugsweise ist die erfindungsgemäße Meßvorrichtung so ausgebildet, daß sie die Messung der Konzentration von Kohlenmonoxid (CO) als Suchstoff im Meßfluid ermöglicht. Dazu wird als Innensensor bevorzugt ein für CO und H₂ empfindlicher CO/H₂-Sensor auf ZrO₂-Basis eingesetzt. Die Anwesenheit von H₂ im Meßfluid führt zu einer Erhöhung der Verpuffungs- bzw. Explosionsgefahr im Meßvolumen. Daher ist die Gasquerempfindlichkeit des Innensensors im Hinblick auf H₂ von Vorteil, denn das H₂ im Meßfluid hat eine Erhöhung des Ausgangssignals des Innensensors zur Folge, so daß die Eignung der erfindungsgemäßen Meßvorrichtung als Warngerät noch verbessert wird. Die Anschlußeinrichtungen derartiger Sensoren können bei Temperaturen von bis zu etwa 150°C zuverlässig arbeiten, wohingegen die sensitiven Sensorbereiche, die für die Anwendungsmöglichkeiten der Sensoren entscheidend sind, deutlich höhere Temperaturen von bis zu 500°C aushalten. Die Ansprechzeit t₉₀ eines derartigen Innensensors ist kürzer als 2 Sekunden.

Für den Außensensor wird vorzugsweise ein amperometrischer Sensor mit einer Ansprechzeit t₉₀ von etwa 30 Sekunden oder mehr verwendet, dessen hohe Meßgenauigkeit bis zu Meßfluid-Temperaturen von etwa 50°C gewährleistet ist.

Die der Erfindung zugrundeliegende Aufgabe wird außerdem erfindungsgemäß im wesentlichen dadurch gelöst, daß bei dem Verfahren zum Betrieb einer gemäß der Erfindung ausgebildeten Meßvorrichtung die Schaltungs- und Auswerteeinheit das Einzelsignal des Innensensors mit Hilfe des genauen Außensensorsignals kalibriert und das kalibrierte Innensensorsignal der Meßwertermittlung zugrunde legt.

Bei diesem Verfahren wird demnach der Meßwert für die Stoffkonzentration lediglich aus dem Signal des schnell ansprechenden Innensensors abgeleitet, während der langsam ansprechende Außensensor nur der Kalibrierung dient und kein direktes Meßsignal liefert. Erfindungsgemäß besteht damit erstmals die Möglichkeit, in einem eine hohe Temperatur aufweisenden und stark mit insbesondere partikelförmigen Verunreinigungen beladenen Meßfluid das zeitliche Verhalten des Absolutwertes der Konzentration eines bestimmten Stoffes im Meßfluid mit hinreichend hoher Genauigkeit wiederzugeben.

Erfindungsgemäß lassen sich somit die Einflüsse langsam veränderlicher Parameter wie beispielsweise Drift des Innensensors sowie Temperatur, Sauerstoffkonzentration, Strömungsgeschwindigkeit des Meßfluids auf den schnell ansprechenden Innensensor beseitigen oder zumindest reduzieren.

Bei entsprechender Ausgestaltung der Schaltungs- und Auswerteeinheit lassen sich mit der gemäß der Erfindung ausgebildeten Meßvorrichtung bei Meßfluid-Temperaturen von bis zu 400°C und Verunreinigungskonzentrationen im Meßfluid von bis zu etwa 100g/m³ innerhalb einer Ansprechzeit von weniger als 2 Sekunden solche Meßwerte der Stoffkonzentration im Meßfluid ermitteln, die 90% des tatsächlichen Wertes mit Abweichungen von weniger als 5% des aktuellen Meßwertes und daher mit einer für die Praxis genügend hohen Genauigkeit repräsentieren.

Ein besonderer Vorteil der Erfindung besteht darin, daß derartige, bisher zumindest unter den vorstehend beschriebenen erschwerten Meßbedingungen unmögliche Meßleistungen erfindungsgemäß auch bei Verwendung von grundsätzlich bekannten Stoffsensoren ermöglicht werden, wie beispielsweise schnell ansprechenden Sensoren auf ZrO₂-Basis und amperometrischen Sensoren mit einer hohen Meßgenauigkeit.

Nach einer besonders bevorzugten Ausgestaltung des erfindungsgemäßen Betriebsverfahrens führt die Schaltungs- und Auswerteeinheit die Kalibrierung des Innensensors in vorzugsweise regelmäßigen Intervallen durch, untersucht dabei jedes Sensorsignal während eines Kalibrierungszeitraumes und korrigiert eine gegebene Innensensor-Kennlinie in Abhängigkeit vom ermittelten Außensensorsignal. Da der prinzipielle Verlauf der Innensensor-Kennlinie bekannt ist, genügt ein einziger mit Hilfe des Außensensors ermittelter Meßwert hoher Genauigkeit, um die Kennlinie des Innensensors gegebenenfalls so zu verschieben, daß auch die vom Innensensor gemessenen Absolutwerte der Stoffkonzentration mit deren tatsächlichem Wert übereinstimmen.

Gemäß einer weiteren Ausführungsform des erfindungsgemäßen Verfahrens werden eventuelle Fehler bei der Innensensor-Kalibrierung vermieden, indem die Schaltungs- und Auswerteeinheit beim Auftreten von schnellen Änderungen der Stoffkonzentration im Kalibrierungszeitraum, die mit Hilfe des schnell ansprechenden Innensensors nachgewiesen werden können, die Kalibrierung abbricht und von neuem beginnt.

Bevorzugt ist die Schaltungs- und Auswerteeinheit des weiteren derart ausgebildet, daß sie die Funktionsfähigkeit der Sensoren entweder ununterbrochen oder in vorzugsweise regelmäßigen Intervallen überprüft und bei Nichtzutreffen festgelegter Kriterien ein Warnsignal ausgibt. Es liegt dann im Ermessen eines Bedieners, gegebenenfalls den den nachzuweisenden Stoff im Meßfluid erzeugenden Prozeß, die gesamte Anlage und / oder eine im Strömungsweg des Meßfluids angeordnete Filteranlage abzuschalten. Grundsätzlich ist auch eine automatische Abschaltung möglich.

Gemäß einer bevorzugten Überprüfungsmethode ermittelt die Schaltungs- und Auswerteeinheit solche Meßwerte aller Sensoren, die das sich langsam ändernde Grundniveau der Stoffkonzentration im Meßfluid repräsentieren, vergleicht diese Meßwerte miteinander und gibt das Warnsignal dann aus, wenn wenigstens eine durch den Vergleich ermittelte Abweichung jeweils zweier Sensor-Meßwerte voneinander einen festgelegten Wert überschreitet. Dies ist beispielsweise dann der Fall, wenn einer der Sensoren ausfällt und falsche Meßwerte liefert.

Eine weitere Ursache für voneinander abweichende Sensor-Einzelmeßwerte können sogenannte "Strähnen" des nachzuweisenden Stoffes im Meßvolumen sein, mit denen vergleisweise hohe, räumlich begrenzte Konzentrationen des nachzuweisenden Stoffes im Meßfluid bezeichnet werden. Voraussetzung für die Erfassung derartiger Stoffsträhnen ist, daß die Sensoren die Messung der Stoffkonzentration jeweils an unterschiedlichen Stellen im Meßvolumen vornehmen. Wenngleich die Existenz derartiger Stoffsträhnen nicht zweifelsfrei bewiesen ist, so ist eine nach dem erfindungsgemäßen Verfahren betriebene Meßvorrichtung doch grundsätzlich dazu in der Lage, diese Strähnen gegebenenfalls nachzuweisen und dann ein Warnsignal und/oder einen Abschaltbefehl auszugeben.

Eine weitere mögliche Überprüfung der Stoffsensoranordnung besteht gemäß einer Weiterbildung des erfindungsgemäßen Verfahrens darin, daß auf eine die Funktion des Innensensors beeinträchtigende Alterung geschlossen wird, wenn durch die Schaltungs- und Auswerteeinheit festgestellt wird, daß die Wiedergabetreue des Innensensors hinsichtlich zeitlicher Änderungen der Stoffkonzentration nicht gewährleistet ist. Dazu untersucht die Schaltungs- und Auswerteeinheit das Ausgangssignal des Innensensors im Hinblick auf vergleichsweise schnelle zeitliche Änderungen der Stoffkonzentration, von denen bekannt ist, daß sie in der Praxis im Meßvolumen tatsächlich auftreten, und die bei genügender Wiedergabetreue des Innensensors daher von dessen Ausgangssignal wiedergespiegelt werden müssen. Beim Fehlen solcher schnellen Stoffkonzentrationsänderungen entsprechenden Anteile im Innensensor-Ausgangssignal findet eine Verschiebung des Frequenzspektrums dieses Ausgangssignals in Richtung tiefer Frequenzen statt. In diesem Fall wird gemäß dem erfindungsgemäßen Verfahren ein Warnsignal ausgegeben.

Eine Überprüfung der Ansprechzeit des Innensensors ist gemäß einer weiteren Ausführungsform des erfindungsgemäßen Verfahrens durch Einblasen von meßfluidfreier Druckluft in die unmittelbare Umgebung des sensitiven Bereiches des Innensensors möglich, wobei das Warnsignal dann ausgegeben wird, wenn sich der durch den Innensensor angezeigte Meßwert für die Stoffkonzentration nicht oder nicht mit einer erwarteten Rate verringert.

Die Schaltungs- und Auswerteeinheit ermöglicht nach einer weiteren Ausgestaltung des erfindungsgemäßen Verfahrens eine Überprüfung des Außensensors dadurch, daß vorzugsweise von einem Bediener dem Außensensor ein Prüfgas zugeführt wird, wobei die Schaltungs- und Auswerteeinheit bevorzugt bei unterhalb eines vorgebbaren Grenzwertes liegenden Abweichungen des Prüfmeßwertes von dem erwarteten Meßwert eine Nachkalibrierung des Außensensors veranlaßt und bei den Grenzwert überschreitenden Abweichungen das Warnsignal ausgibt.

Außerdem kann dem Außensensor mittels der Luftzufuhranordnung Umgebungsluft zugeführt werden, die im wesentlichen frei von Meßfluid ist, so daß auf diese Weise eine Überprüfung des Nullpunkts des Außensensors gestattet wird.

Durch die vorstehend beschriebenen zahlreichen Überwachungsmöglichkeiten, zu denen die Schaltungs- und Auswerteeinheit der gemäß der Erfindung ausgebildeten Meßvorrichtung bei Anwendung des erfindungsgemäßen Betriebsverfahrens in der Lage ist, wird die Zuverlässigkeit der Meßvorrichtung sowie der von ihr ermittelten Absolutwerte für die Stoffkonzentration beträchtlich erhöht.

Weitere vorteilhafte Ausführungsformen sowohl der erfindungsgemäßen Meßvorrichtung als auch des erfindungsgemaßen Verfahrens zum Betrieb dieser Meßvorrichtung sind in den Unteransprüchen angegeben.

Die Erfindung wird im folgenden beispielhaft anhand der Zeichnung beschrieben; in dieser zeigt:
- Figur 1: eine Ausführungsform einer gemäß der Erfindung ausgebildeten Meßvorrichtung, die zwei in einem Meßvolumen angeordnete Innensensoren und einen außerhalb des Meßvolumens vorgesehenen Außensensor umfaßt,
- Figur 2: einen Schnitt durch eine Ausführungsform eines in Verbindung mit dem Außensensor einer erfindungsgemäßen Meßvorrichtung verwendeten Kondensatabscheiders, und
- Figur 3: gleichzeitig drei Teilschnitte durch den Kondensatabscheider von Figur 2 gemäß den Linien A-A, B-B bzw. C-C.

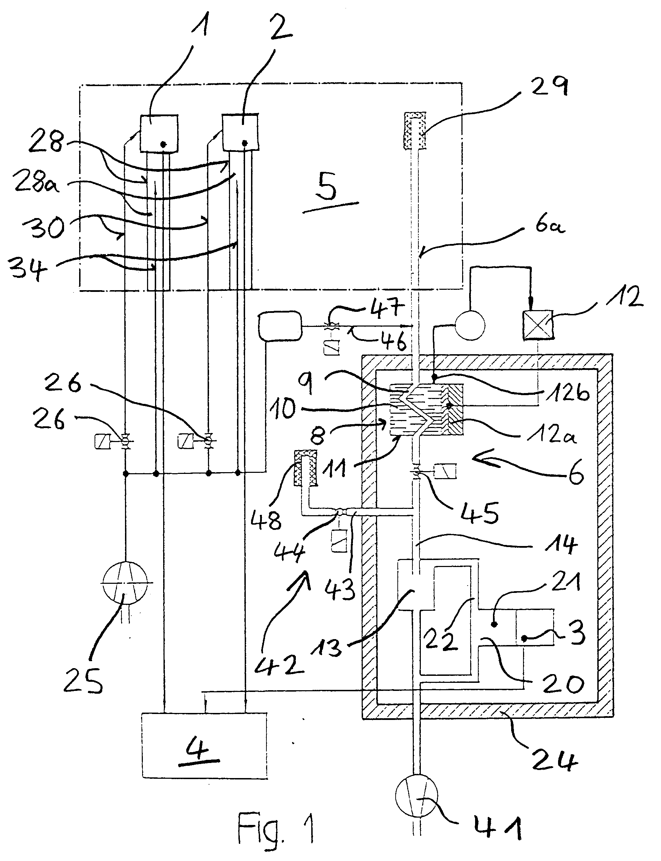

Nach Figur 1, in der eine Ausführungsform einer erfindungsgemäßen Meßvorrichtung dargestellt ist, sind in einem Meßvolumen 5, das von einem Meßfluid durchströmt wird, zwei Innensensoren 1, 2 sowie der Ansaugfilter 29 eines außerhalb des Meßvolumens 5 befindlichen Außensensors 3 angeordnet. Das Meßfluid im Meßvolumen 5 enthält in einer bestimmten Konzentration einen Suchstoff, der mittels der Sensoren 1, 2, 3 nachweisbar ist.

Die Meßsignale der Innensensoren 1, 2 werden einer außerhalb des Meßvolumens 5 angeordneten Schaltungs- und Auswerteeinheit 4 zugeführt, die auch das Ausgangssignal des Außensensors 3 empfängt und aus den empfangenen Einzelsignalen der Sensoren 1, 2, 3 den momentanen Wert der Stoffkonzentration im Meßfluid ableitet.

Mit dem im Meßvolumen 5 befindlichen Ansaugfilter 29 des Außensensors 3 ist ein extraktiver Meßkanal 6 gekoppelt, über den ein angesaugter Meßfluidteilstrom dem Außensensor 3 zur Messung der Stoffkonzentration zuführbar ist. Der Ansaugfilter 29 sorgt dafür, daß das dem Außensensor 3 zugeführte Meßfluid weitgehend von Verunreinigungen befreit ist.

Vom Ansaugfilter 29 bis außerhalb des Meßvolumens 5 ist der Meßkanal 6 im wesentlichen von einem Entnahmerohr 6a gebildet. Im Anschluß an das außerhalb des Meßvolumens 5 gelegene Ende des Entnahmerohres 6a verläuft der Meßkanal 6 teilweise durch ein vorzugsweise mit als Kühlfluid 10 dienendem Wasser gefülltes Kühlgefäß 11. Vorzugsweise ist der Meßkanal 6 im Bereich des Kühlgefäßes 11 als eine sich spiralförmig in vertikaler Richtung erstreckende und vom Meßfluid abwärts durchströmte Kühlrohrschlange 9 ausgebildet. Der durch das Kühlgefäß 11 verlaufende Teil des Meßkanals 6 und das mit dem Kühlfluid 10 gefüllte Kühlgefäß 11 bilden einen Gaskühler 8, durch welchen das dem Außensensor 3 zuzuführende Meßfluid auf eine Temperatur abkühlbar ist, die für eine Messung der Stoffkonzentration im Meßfluid durch den Außensensor 3 mit hoher Genauigkeit erforderlich ist.

Das Kühlgefäß 11 ist mittels eines Regelorgans 12 temperaturregelbar, wobei das Regelorgan 12 zu diesem Zweck ein in einer Seitenwand des Kühlgefäßes 11 angeordnetes Peltierelement 12a sowie einen Temperatursensor 12b umfaßt, der vorzugsweise in das Kühlfluid 10 im Kühlgefäß 11 eingetaucht ist. Dem Temperatursensor 12b kann bevorzugt eine Solltemperatur vorgegeben werden, auf welcher das Kühlfluid 10 gehalten werden soll.

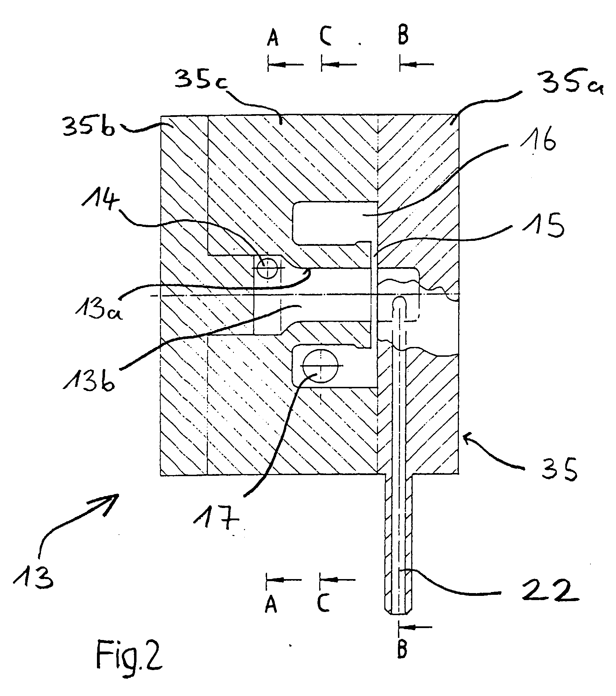

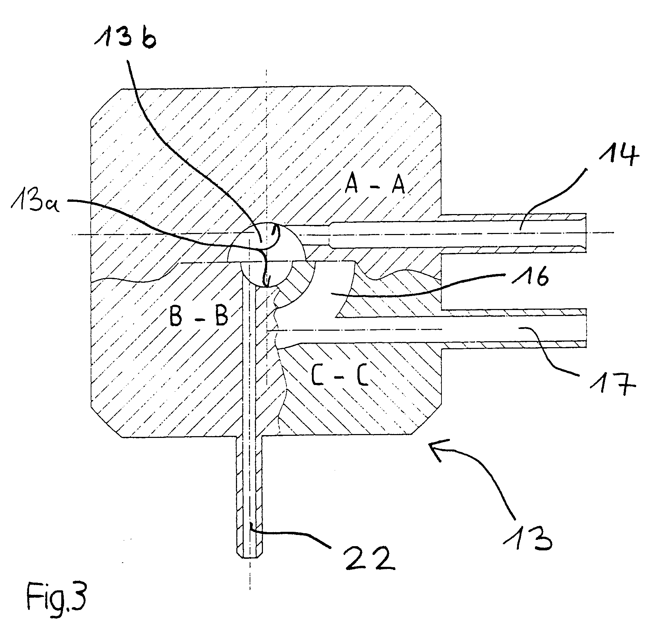

Ausgehend von der Unterseite des Kühlgefäßes 11 mündet der Meßkanal 6 in einen im Bereich des Außensensors 3 angeordneten Kondensatabscheider 13, der eine Drallkammer umfaßt, welche aus dem dem Außensensor 3 zugeführten Meßfluidteilstrom Kondensate abscheidet, die sonst eine Messung der Stoffkonzentration mittels des Außensensors 3 beeinträchtigen würden. Der Aufbau des Kondensatabscheiders 13 und der Drallkammer wird an anderer Stelle anhand der Figuren 2 und 3 beschrieben.

Der Kondensatabscheider 13 weist einen Meßauslaß und einen Kondensatauslaß auf. Vom Meßauslaß führt eine Bypassleitung 22 zunächst zu einem Sensorvorraum 20, an dessen einer Stirnseite die Bypassleitung 22 einmündet. An die andere Stirnseite des Sensorvorraumes 20 schließt sich der Außensensor 3 an. Im Sensorvorraum 20 ist ein PTC-Element 21 angeordnet, durch welches eine Temperaturregelung des Sensorvorraumes 20 ermöglicht wird. Vom Sensorvorraum 20 führt die Bypassleitung 22 weiter zu einer Abführleitung 17, die mit dem Kondensatauslaß des Kondensatabscheiders 13 verbunden ist.

Die Bypassleitung 22 dient dazu, während des normalen Meßbetriebs dem Außensensor 3 über den Sensorvorraum 20 ein nahezu trockenes, von Kondesat weitgehend befreites Meßfluid zuzuführen, während das in der Drallkammer des Kondensatabscheiders 13 aus dem Meßfluid entfernte Kondensat über die Abführleitung 17 nach außen abtransportiert wird. Mittels des PTC-Elements 21 ist das im wesentlichen trockene Meßfluid im Sensorvorraum 20 vor der Messung durch den Außensensor 3 vorzugsweise auf wenige Grad über den Taupunkt aufheizbar.

Der Außensensor 3, der Kondensatabscheider 13 mit Bypassleitung 22 und Sensorvorraum 20 sowie der Gaskühler 8 befinden sich im Inneren eines wärmeisolierten Außengehäuses 24.

Stromabwärts der Wiedereinmündung der Bypassleitung 22 in die Abführleitung 17 tritt die Abführleitung 17 durch das Außengehäuse 24 nach außen und ist außerhalb des Gehäuses 24 an eine Gasfördereinrichtung 41 angeschlossen, die bevorzugt als mit Druckluft betreibbarer Strahlapparat ausgebildet ist.

Der Meßkanal 6 ist des weiteren mit einer Luftzufuhranordnung 42 versehen. Die Luftzufuhranordnung 42 umfaßt eine Luftzufuhrleitung 43, die innerhalb des Gehäuses 24 und zwischen dem Gaskühler 8 und dem Kondensatabscheider 13 in den Meßkanal 6 mündet und ein Ventil 44 aufweist. Das freie, außerhalb des Gehäuses 24 gelegene Ende der Luftzufuhrleitung 43 ist mit einem Luftfilter 48 versehen. Die Luftzufuhranordnung 42 umfaßt außerdem ein weiteres Ventil 45, das zwischen dem Gaskühler 8 und der Mündung der Luftzufuhrleitung 43 im Meßkanal 6 angeordnet ist.

Während des normalen Betriebs saugt die Gasfördereinrichtung 41 über den Meßkanal 6 bei geöffnetem Ventil 45 und geschlossenem Ventil 44 aus dem Meßvolumen 5 den Meßfluidteilstrom an, der dem Außensensor 3 zur Messung der Stoffkonzentration zugeführt wird.

Wenn das im Meßkanal 6 angeordnete Ventil 45 geschlossen und das Ventil 44 in der Luftzufuhrleitung 43 geöffnet ist, sorgt die Gasfördereinrichtung 41 dafür, daß durch die Luftzufuhrleitung 43 über den Luftfilter 48 gereinigte Umgebungsluft dem Außensensor 3 zugeführt wird. Die Gasfördereinrichtung 41 kann daher sowohl während des normalen Meßbetriebs als auch während der Erholungsphasen, in denen die Anordung aus Kondensatabscheider 13, Sensorvorraum 20 und Außensensor 3 mit im wesentlichen meßfluidfreier Umgebungsluft gespült wird, ununterbrochen betrieben werden. Der Wechsel zwischen Meß- und Spülbetrieb erfordert daher lediglich ein Umschalten der Ventile 44 und 45.

Der Kondensatabscheider 13, die Bypassleitung 22 und der Sensorvorraum 20 sind dabei derart ausgebildet und angeordnet, daß eine einwandfreie Spülung des Außensensors 3 mittels der Luftzufuhranordnung 42 gewährleistet ist.

Dem Meßkanal 6 ist ferner eine Druckluftleitung 46 zugeordnet, in der ein Ventil 47 angeordnet ist und an die der Meßkanal 6 außerhalb sowohl des Gehäuses 24 als auch des Meßvolumens 5 angeschlossen ist. Über eine bevorzugt als Membrankompressor ausgebildete Druckluftquelle 25 ist der Druckluftleitung 46 bei geöffnetem Ventil 47 Druckluft zuführbar, um bei geschlossenem Ventil 45 der Luftzufuhranordnung 42 den Ansaugfilter 29 des Meßkanals 6 von Verunreinigungen zu befreien. Dieses Freiblasen des Ansaugfilters 29 erfolgt bevorzugt periodisch und vorzugsweise während der Spül- und Erholungsphasen des Außensensors 3, in denen das Ventil 45 bereits geschlossen ist.

Die Ventile 44, 45 und 47 sind bevorzugt als Schalt- bzw. Magnetventile ausgebildet.

Den Innensensoren 1, 2 der erfindungsgemäßen Meßvorrichtung ist jeweils eine an einer Begrenzungswand des Meßvolumens 5 befestigte Tauchsonde 28 zugeordnet, die als mechanische Halterung für den betreffenden Innensensor 1, 2 dient. Der überwiegende Teil der Innensensoren 1, 2 befindet sich dabei jeweils im Inneren ihrer Tauchsonde 28. Die sensitiven Bereiche der Innensensoren 1, 2 sind jedoch außerhalb der Tauchsonden 28 angeordnet, so daß sie direkt vom das Meßvolumen 5 durchströmenden Meßfluid umspült werden können.

Abweichend von der Darstellung in Figur 1 können die Eintauchtiefen der sensitiven Bereiche der Innensenoren 1, 2 in das Meßvolumen 5 verschieden sein und sich jeweils auch von derjenigen des Ansaugfilters 29 unterscheiden. Auf diese Weise wäre die Stoffkonzentration im Meßfluid an mehreren, unterschiedliche Abstände von den Begrenzungswänden des Meßvolumens 5 aufweisenden Stellen im Meßvolumen 5 meßbar.

Das Innere der Tauchsonden 28 bildet jeweils einen fluiddicht vom Meßvolumen 5 abgetrennten Anschlußraum 28a, in welchem die temperaturempfindlichen Teile des jeweiligen Innensensors 1, 2, insbesondere dessen mit den nach außen führenden Signalleitungen gekoppelten Anschlußeinrichtungen vor dem heißen Meßfluid geschützt untergebracht sind. In die Anschlußräume 28a mündet jeweils eine Druckluftleitung 34, über die dem jeweiligen Anschlußraum 28a von außen durch den Kompressor 25 Druckluft zuführbar ist, um den Anschlußraum 28a gegebenenfalls auf eine für die empfindlichen Teile des jeweiligen Innensensors 1, 2 unkritische Temperatur kühlen zu können.

Darüber hinaus weist jeder Innensensor 1, 2 eine nicht dargestellte Temperaturregelung auf, die bevorzugt eine den jeweiligen Innensensor 1, 2 auf eine geeignete Betriebstemperatur erwarmende Heizeinrichtung umfaßt. Dabei werden vorzugsweise jeweils die Innenwiderstände der Innensensoren 1, 2 zur Messung der Innensensor-Temperatur verwendet. Auf diese Weise können die Innensensoren 1, 2 an die Temperatur und die Strömungsgeschwindigkeit des Meßfluids, welche die Wärmeübertragung vom Meßfluid auf die Innensensoren 1, 2 beeinflußt, angepaßt werden. Die Aufrechterhaltung der optimalen Arbeitstemperatur der Innensensoren 1, 2 wird dadurch ermöglicht.

Jeder Innensensor 1, 2 ist ferner mit einer weiteren, sich außen am jeweiligen Tauchsensor 28 entlang erstreckenden Druckluftleitung 30 versehen, die ebenfalls von außerhalb des Meßvolumens 5 mittels des Kompressors 25 mit Druckluft versorgbar ist, wobei die Druckluftzufuhr mittels außerhalb des Meßvolumens 5 in den betreffenden Luftleitungen angeordneter Ventile 26 regelbar ist, die ebenfalls bevorzugt als Schalt- bzw. Magnetventile ausgebildet sind.

Die Druckluftleitungen 30 enden vorzugsweise nahe dem jeweiligen sensitiven Bereich der Innensensoren 1, 2 und sind dort jeweils mit einer Reinigungsdüse versehen, die auf den betreffenden sensitiven Innensensorbereich gerichtet ist, so daß mittels gerichteter Druckluftstöße sich auf den sensitiven Bereichen abgelagerte Verunreinigungen gezielt weggeblasen werden können.

Figur 2 zeigt einen Schnitt längs einer senkrecht zum in Figur 1 gezeigten Eintrittsstutzen 14 verlaufenden Ebene durch ein die Drallkammer 13b umgebendes Gehäuse 35 des Kondensatabscheiders 13.

Die einen vom Gehäuse 35 umgebenden Hohlraum darstellende Drallkammer 13b wird von einer rotationssymmetrischen Innenwand 13a begrenzt und weist im Bereich der Einmündung des Eintrittsstutzens 14 eine vergrößerte Querschnittsfläche auf. Im Richtung der Bypassleitung 22 geht die Drallkammer 13b in einen zylindrischen Bereich mit einer kleineren Querschnittsfläche über. Bezüglich der Drallkammer-Längsrichtung etwa mittig im zylindrischen Bereich ist die Drallkammer 13b mit einem Umfangsschlitz 15 versehen, der die Drallkammer 13b mit einem Ringraum 16 verbindet. Der Ringraum 16 umgibt die Drallkammer 13b konzentrisch und kommuniziert mit der Abführleitung 17.

Die Gehäusewand 35a, welche die Drallkammer 13b an demjenigen Ende begrenzt, in dessen Bereich die Bypassleitung 22 einmündet, der die Drallkammer 13b sowie den Ringraum 16 in radialer Richtung begrenzende Gehäuseteil 35c sowie die Gehäusewand 35b, welche die Drallkammer 13b an ihrem anderen Ende begrenzt, sind als separate Gehäuseteile ausgebildet, die mittels nicht dargestellter Verbindungselemente 36 lösbar miteinander verbunden sind. Die Drallkammer 13b ist daher zerlegbar, wodurch die Drallkammer 13b, der Ringraum 16 sowie der Mündungsbereich der Bypassleitung 22 beispielsweise zu Reinigungszwecken auf einfache Weise von außen zugänglich sind.

Figur 3 zeigt gleichzeitig drei Teilschnitte durch den Kondensatabscheider 13 von Figur 2 jeweils in einer parallel zu den Stirnseiten der Drallkammer 13b verlaufenden Ebene gemäß den Schnittlinien A-A, B-B und C-C in Figur 2.

Zu erkennen sind in Figur 3 der in den Bereich größerer Querschnittsfläche der Drallkammer 13b mündende Eintrittsstutzen 14 sowie der Ringraum 16, der den Bereich kleinerer Querschnittsfläche der Drallkammer 13b konzentrisch umgibt. Die die Drallkammer 13b begrenzende Innenwand 13a besitzt in jeder Ebene senkrecht zur Drallkammerlängsachse einen kreisförmigen Querschnitt.

Die mit dem Ringraum 16 kommunizierende Abführleitung 17 erstreckt sich parallel versetzt zum Eintrittsstutzen 14, wobei sich sowohl der Eintrittsstutzen 14 als auch die Abführleitung 17 tangential zur Drallkammer 13b erstrecken, so daß durch den Eintrittsstutzen 14 in die Drallkammer 13b einströmendes Meßfluid im Inneren der Drallkammer 13b deren Längsachse schraubenförmig umströmt. Dabei nimmt die Strömungsgeschwindigkeit des Meßfluids aufgrund des sich in Richtung der Bypassleitung 22 verkleinernden Drallkammerquerschnitts noch weiter zu, und die auf diese Weise wirkenden Zentrifugalkräfte sorgen für eine Abscheidung von Kondensaten aus dem Meßfluid, welche über den in Figur 2 erkennbaren Umfangsschlitz 15 und den Ringraum 16 durch die Abführleitung 17 nach außen abgeführt werden. Dadurch ist gewährleistet, daß durch die Bypassleitung 22 ein trockenes, von Kondensat weitgehend befreites Meßfluid in den Sensorvorraum 20 gelangt und für die Messung der Stoffkonzentration durch den Außensensor 3 zur Verfügung steht. Das PTC-Element 21 sorgt dabei für optimale Temperaturverhältnisse während der Messungen.

Die Funktionsweise der erfindungsgemäßen Meßvorrichtung, die vorzugsweise nach dem Verfahren der Erfindung betrieben wird, ist wie folgt:

Während des Meßbetriebs liefern die schnell ansprechenden Innensensoren 1, 2 ständig Meßsignale an die Schaltungs- und Auswerteeinheit 4. Vorzugsweise werden als Innensensoren 1, 2 für Kohlenmonoxid und H₂ empfindliche CO/H₂-Sensoren auf ZrO₂-Basis verwendet, die eine Ansprechzeit t₉₀ von weniger als 2 Sekunden besitzen, so daß die momentane Stoffkonzentration im das Meßvolumen 5 durchströmenden Meßfluid praktisch verzögerungsfrei durch die Innensensoren 1, 2 gemessen wird.

Über den im Inneren des Meßvolumens 5 mit dem Ansaugfilter 29 versehenen Meßkanal 6 wird dem Außensensor 3 ein Meßfluidteilstrom zugeführt, der durch das Vorsehen des Ansaugfilters 29 und des Gaskühlers 8 sowie der Drallkammer 13b weitgehend von partikelförmigen Verunreinigungen sowie von Kondensaten befreit und auf eine Temperatur abgekühlt ist, die für die Messung der Stoffkonzentration mittels eines vorzugsweise von einem amperometrischen Sensor gebildeten Außensensors 3 geeignet ist, dessen hohe Meßgenauigkeit bis zu Meßfluid-Temperaturen von etwa 50°C gewährleistet ist. Ein derartiger Sensor weist eine Ansprechzeit t₉₀ von etwa 30 Sekunden oder mehr auf, so daß mit dem Außensensor 3 kurzzeitige Änderungen der Stoffkonzentration im Meßfluid zwar nicht erfaßt werden können, jedoch das sich langsam ändernde Grundniveau der Stoffkonzentration dagegen mit hoher Genauigkeit gemessen werden kann.

Die Schaltungs- und Auswerteeinheit 4 der erfindungsgemäßen Meßvorrichtung verwendet den vom Außensensor 3 gelieferten Meßwert hoher Genauigkeit dazu, die bekannte Kennlinie der Innensensoren 1, 2 zu kalibrieren, indem sie in regelmäßigen Zeitabständen für jedes Einzelsignal der Sensoren 1, 2, 3 den zeitlichen Mittelwert über einen Kalibrierungszeitraum bildet und dann gegebenenfalls jede Innensensor-Kennlinie in Abhängigkeit von dem Mittelwert des Außensensors 3 korrigiert. Vorzugsweise wird etwa alle 30 Minuten eine Kalibrierung von etwa 5 Minuten Dauer durchgeführt, wobei diese Zeiten bevorzugt auf beliebige Werte einstellbar sind.

Die Kalibrierung wird vorzugsweise dann abgebrochen und erneut begonnen, wenn während des Kalibrierungszeitraumes schnelle Änderungen der Stoffkonzentration auftreten, die mittels der Innensensoren 1, 2 nachweisbar sind, da in einem derartigen Fall die von dem schnell ansprechenden Innensensor und dem langsam ansprechenden Außensensor gelieferten Meßwerte zwangsläufig voneinander abweichen und somit zur Kalibrierung nicht herangezogen werden können.

Durch die erfindungsgemäße Meßvorrichtung wird es somit in vorteilhafter Weise erstmals ermöglicht, in Umgebungen, die durch hohe Temperaturen und starke Belastungen mit insbesondere partikelförmigen Verunreinigungen charakterisiert sind, das zeitliche Verhalten der Konzentration eines bestimmten Stoffes wie beispielsweise Kohlenmonoxid mit großer Genauigkeit zu messen, wobei nicht lediglich das Vorhandensein von zeitlichen Stoffkonzentrationsschwankungen nachweisbar, sondern auch der Absolutwert der Stoffkonzentration in jedem Moment genau meßbar ist.

In vorzugsweise regelmäßigen Abständen erfolgt eine Abreinigung der sensitiven Bereiche der Innensensoren 1, 2 mittels der über die Druckluftleitungen 30 mit Druckluft beaufschlagbaren Reinigungsdüsen, wobei die Schaltungs- und Auswerteeinheit 4 sicherstellt, daß zu jedem Zeitpunkt wenigstens ein Innensensor 1, 2 nicht abgereinigt wird und weiter Meßsignale liefert. Die ununterbrochene Erfassung schneller Änderungen der Stoffkonsentration im Meßfluid ist dadurch gewährleistet.

Ebenso erfolgt in bevorzugt regelmäßigen Intervallen eine manuell oder automatisch veranlaßte Spülung des Außensensors 3 mittels der Luftzufuhranordnung 42, um Erholungsphasen für den Außensensor 3 zu schaffen, die zur Aufrechterhaltung von dessen hoher Meßgenauigkeit notwendig sind. Die dazu erforderliche, oben beschriebenen Steuerung der in der Druckluftleitung 43 und dem Meßkanal 6 angeordneten Ventile 44 und 45 kann dabei durch die Schaltungs- und Auswerteeinheit 4 erfolgen.

Die Zuverlässigkeit und Meßgenauigkeit der erfindungsgemäßen Meßvorrichtung kann weiter dadurch erhöht werden, daß die Schaltungs- und Auswerteeinheit 4 die Funktionsfähigkeit der Sensoren 1, 2, 3 ununterbrochen bzw. in bevorzugt regelmäßigen Intervallen überprüft und bei Nichtzutreffen festgelegter Kriterien ein Warnsignal ausgibt. Zu den zahlreichen Überprüfungsmöglichkeiten, die nach dem erfindungsgemäßen Verfahren zum Betrieb der Meßvorrichtung der Erfindung möglich sind, wird auf die entsprechenden Ansprüche sowie auf den Einleitungsteil der Beschreibung verwiesen.

### Bezugszeichenliste

- 1, 2: Innensensoren
- 3: Außensensor
- 4: Schaltungs- und Auswerteeinheit
- 5: Meßvolumen
- 6: Meßkanal
- 6a: Entnahmerohr
- 8: Gaskühler
- 9: Kühlrohrschlange
- 10: Kühlfluid
- 11: Kühlgefäß
- 12: Regelorgan
- 12a: Peltierelement
- 12b: Temperatursensor
- 13: Kondensatabscheider
- 13a: Innenwand
- 13b: Drallkammer
- 14: Eintrittsstutzen
- 15: Schlitz
- 16: Ringraum
- 17: Abführleitung
- 20: Sensorvorraum
- 21: PTC-Element
- 22: Bypassleitung
- 24: Außengehäuse
- 25: Kompressor
- 26: Ventile
- 28: Tauchsonden
- 28a: Anschlußraum
- 29: Ansaugfilter
- 30: Druckluftleitungen
- 34: Druckluftleitung
- 35: Gehäuse
- 35a,b: Seitenwände
- 35c: Umfangswand
- 41: Gasfördereinrichtung
- 42: Luftzufuhranordnung
- 43: Luftzufuhrleitung
- 44: Ventil
- 45: Ventil
- 46: Druckluftleitung
- 47: Ventil
- 48: Luftfilter

## Patentansprüche

1. Meßvorrichtung zur Messung der Konzentration eines bestimmten Stoffes in einem von einem Meßfluid durchströmten Meßvolumen, insbesondere der Kohlenmonoxid-Konzentration in einem heißen, staubbeladenen Abgas,
**gekennzeichnet** durch
eine Stoffsensoranordnung mit wenigstens einem schnell ansprechenden Innensensor (1, 2) mit Kurzzeitstabilität innerhalb des Meßvolumens (5) und wenigstens einem langsam ansprechenden Außensensor (3) hoher Meßgenauigkeit außerhalb des Meßvolumens (5), und eine mit den Sensoren (1, 2, 3) verbundene Schaltungs- und Auswerteeinheit (4), welche die Einzelsignale der Sensoren (1, 2, 3) empfängt und aus den empfangenen Einzelsignalen einen den momentanen Wert der Stoffkonzentration im Meßfluid mit hoher Genauigkeit repräsentierenden Meßwert ableitet.

2. Meßvorrichtung nach Anspruch 1,
dadurch **gekennzeichnet,**
daß der langsam ansprechende Außensensor (3) mit dem Meßvolumen (5) durch einen extraktiven Meßkanal (6) verbunden ist, über den ein Meßfluidteilstrom dem Meßvolumen (5) entnehmbar und dem Außensensor (3) zur genauen Messung der Stoffkonzentration zuführbar ist, wobei der Meßkanal (6) ein Entnahmerohr (6a), dessen im Meßvolumen (5) gelegenes Ende mit einem Ansaugfilter (29) versehen ist, eine Gasfördereinrichtung (41), einen Gaskühler (8) und/oder einen Kondensatabscheider (13) umfaßt, wobei insbesondere der Meßkanal (6) mit einer Luftzufuhranordnung (42) versehen ist, die eine vorzugsweise periodische Beaufschlagung des Außensensors (3) mit Umgebungsluft gestattet und vorzugsweise die Luftzufuhranordnung (42) eine Luftzufuhrleitung (43), die stromaufwärts des Kondensatabscheiders (13) in den Meßkanal (6) mündet und mit einem Ventil (44) versehen ist, sowie ein weiteres Ventil (45) umfaßt, welches im Meßkanal (6) stromaufwärts der Mündung der Luftzufuhrleitung (43) angeordnet ist.

3. Meßvorrichtung nach Anspruch 2,
dadurch **gekennzeichnet,**
daß dem Meßkanal (6) zum vorzugsweise periodischen Freiblasen des Ansaugfilters (29) eine mit einem Ventil (47) versehene Druckluftleitung (46) zugeordnet ist, welche bevorzugt stromaufwärts des im Meßkanal (6) angeordneten Ventils (45) der Luftzufuhranordnung (42) in den Meßkanal (6) mündet, und/oder daß eine Bypassleitung (22) für ein trockenes, von Kondensat im wesentlichen befreites Meßfluid von einem Meßauslaß des Kondensatabscheiders (13) zum Außensensor (3) und von dort weiter zur Gasfördereinrichtung (41) führt, und daß ein Kondensatauslaß des Kondensatabscheiders (13) durch eine Abführleitung (17) für einen das abgeschiedene Kondensat transportierenden Teilgasstrom mit der Gasfördereinrichtung (41) direkt verbunden ist, wobei insbesondere der Kondensatabscheider (13) eine Drallkammer (13b) umfaßt, die als im wesentlichen rotationssymmetrischer, teilweise zylindrischer Hohlraum ausgebildet ist, in den an seinem einen Ende ein vorzugsweise tangentialer Eintrittsstutzen (14) für den Meßfluidteilstrom mündet, wobei der Meßauslaß am anderen Ende des Hohlraums angeordnet und in der den Hohlraum begrenzenden Innenwand (13a) ein umlaufender Schlitz (15) vorgesehen ist, der den Hohlraum mit einem im wesentlichen konzentrischen Ringraum (16) verbindet, an den sich die Abführleitung (17) anschließt, die sich bevorzugt tangential zur Drallkammer (13b) und in der durch den Eintrittsstutzen (14) vorgegebenen Drallrichtung erstreckt.

4. Meßvorrichtung nach einem der Ansprüche 2 oder 3,
dadurch **gekennzeichnet,**
daß die Gasfördereinrichtung (41) als mit Druckluft betreibbarer Strahlapparat ausgebildet ist, und/oder daß sich die Bypassleitung (22) im Bereich des Außensensors (3) zu einem Sensorvorraum (20) erweitert, in welchem vorzugsweise ein PTC-Element (21) angeordnet ist, welches eine Aufheizung des Meßfluids auf eine Temperatur oberhalb des Taupunktes gestattet und/oder daß der Gaskühler (8) eine bevorzugt sich im wesentlichen vertikal erstreckende und vom Meßfluid abwärts durchströmte Kühlrohrschlange (9) umfaßt, die in einem temperaturregelbaren und mit einem Kühlfluid (10), insbesondere Wasser gefüllten Kühlgefäß (11) angeordnet ist, wobei insbesondere zur Temperaturregelung des Kühlgefäßes (11) ein ein Peltierelement (12a) und einen Temperatursensor (12b) umfassendes Regelorgan (12) vorgesehen ist.

5. Meßvorrichtung nach einem der Ansprüche 2 bis 4,
dadurch **gekennzeichnet,**
daß der Außensensor (3), der Kondensatabscheider (13) und der Gaskühler (8) in einem wärmeisolierten Außengehäuse (24) untergebracht sind.

6. Meßvorrichtung nach einem der vorhergehenden Ansprüche,
dadurch **gekennzeichnet,**
daß zur mechanischen Aufnahme des im Meßvolumen (5) befindlichen Innensensors (1, 2) eine Tauchsonde (28) vorgesehen ist, die eine direkte Umspülung zumindest des sensitiven Bereiches des Innensensors (1, 2) mit dem Meßfluid ermöglicht, wobei insbesondere die Tauchsonde (28) zur Unterbringung temperaturempfindlicher Teile des Innensensors (1, 2) einen fluiddicht vom Meßvolumen (5) abgetrennten Anschlußraum (28a) aufweist, der bevorzugt mittels von außen zuführbarer Druckluft auf eine Temperatur von vorzugsweise weniger als etwa 150°C kühlbar ist.

7. Meßvorrichtung nach einem der vorhergehenden Ansprüche,
dadurch **gekennzeichnet,**
daß wenigstens eine mit einer Druckluftleitung (30) gekoppelte Reinigungsdüse vorgesehen ist, die mittels gerichteter Druckluftstöße eine vorzugsweise zyklische Abreinigung des sensitiven Bereiches des Innensensors (1, 2) ermöglicht, und/oder daß zur Einhaltung der Arbeitstemperatur des Innensensors (1, 2) eine die Innensensor-Temperatur an momentane Charakteristiken des Meßfluids im Meßvolumen (5), insbesondere an die Temperatur und die Strömungsgeschwindigkeit des Meßfluids anpassende Regeleinrichtung vorgesehen ist, die vorzugsweise eine den Innensensor (1, 2) auf eine geeignete Betriebstemperatur erwärmende Heizeinrichtung umfaßt und bevorzugt die Temperaturabhängigkeit des Innenwiderstandes des Innensensors (1, 2) zur Messung der Innensensor-Temperatur verwendet.

8. Meßvorrichtung nach einem der vorhergehenden Ansprüche,
dadurch **gekennzeichnet,**
daß wenigstens zwei schnell ansprechende Innensensoren (1, 2) mit Kurzzeitstabilität vorgesehen sind, wobei insbesondere zumindest die sensitiven Bereiche der Innensensoren (1, 2) unterschiedlich weit in das Meßvolumen (5) eingetaucht sind und sich vorzugsweise die Eintauchtiefe des mit dem Ansaugfilter (29) versehenen Endes des Außensensor-Meßkanals (6) von den Innensensor-Eintauchtiefen unterscheidet, und/oder daß der Innensensor (1, 2) als ein für CO und H₂ empfindlicher CO/H₂-Sensor auf ZrO₂-Basis mit einer Ansprechzeit t₉₀, nach der eine Anzeige von 90% des End-Meßwertes erfolgt, von weniger als 2 Sekunden ausgebildet ist, wobei der sensitive Bereich des Sensors (1, 2) mit Meßfluid-Temperaturen von bis zu etwa 500°C belastbar ist und die Anschlußeinrichtungen des Sensors (1, 2) bei Temperaturen bis zu etwa 150°C funktionsfähig sind, und/oder daß der Außensensor (3) als ein amperometrischer Sensor mit einer Ansprechzeit t₉₀, nach der eine Anzeige von 90% des End-Meßwertes erfolgt, von wenigstens etwa 30 Sekunden ausgebildet ist, wobei die Meßgenauigkeit des Außensensors (3) bis zu Meßfluid-Temperaturen von etwa 50°C gewährleistet ist.

9. Verfahren zum Betrieb einer Meßvorrichtung nach einem der vorhergehenden Ansprüche,
dadurch **gekennzeichnet,**
daß die Schaltungs- und Auswerteeinheit (4) das Signal des Innensensors (1, 2) mit Hilfe des genauen Außensensorsignals kalibriert und das kalibrierte Innensensorsignal der Meßwertermittlung zugrunde legt.

10. Verfahren nach Anspruch 9,
dadurch **gekennzeichnet,**
daß die Schaltungs- und Auswerteeinheit (4) die Kalibrierung des Innensensors (1, 2) in vorzugsweise regelmäßigen Intervallen durchführt und dazu für jedes Sensorsignal den zeitlichen Mittelwert über einen Kalibrierungszeitraum bildet und eine gegebene Innensensor-Kennlinie in Abhängigkeit von dem Außensensorsignal korrigiert, wobei insbesondere die Schaltungs- und Auswerteeinheit (4) beim Auftreten von schnellen Änderungen der Stoffkonzentration im Kalibrierungszeitraum die Kalibrierung abbricht und erneut beginnt.

11. Verfahren nach einem der Ansprüche 9 oder 10,
dadurch **gekennzeichnet,**
daß die Schaltungs- und Auswerteeinheit (4) die Abreinigung der Innensensoren (1, 2) derart steuert, daß zu jedem Zeitpunkt wenigstens ein Innensensor (1, 2) Meßsignale liefert, so daß eine ununterbrochene Erfassung von schnellen Änderungen der Stoffkonzentration sichergestellt ist, und/oder daß die Schaltungs- und Auswerteeinheit (4) die Funktionsfähigkeit der Sensoren (1, 2, 3) ununterbrochen oder in bevorzugt regelmäßigen Intervallen überprüft und bei Nichtzutreffen festgelegter Kriterien ein Warnsignal ausgibt, wobei insbesondere die Schaltungs- und Auswerteeinheit (4) die das sich langsam ändernde Grundniveau der Stoffkonzentration repräsentierenden Meßwerte der einzelnen Sensoren (1, 2, 3) miteinander vergleicht und das Warnsignal dann ausgibt, wenn wenigstens eine durch den Vergleich ermittelte Abweichung jeweils zweier Sensor-Meßwerte voneinander einen Wert überschreitet, der insbesondere durch eine den Normalzustand darstellende Streubreite der Sensor-Meßwerte festgelegt ist.

12. Verfahren nach Anspruch 11,
dadurch **gekennzeichnet,**
daß die Schaltungs- und Auswerteeinheit (4) zur Überprüfung der Funktionsfähigkeit des Innensensors (1, 2) die Wiedergabetreue des Innensensors (1, 2) hinsichtlich zeitlicher Änderungen der Stoffkonzentration überwacht und auf eine die Funktion des Innensensors (1, 2) beeinträchtigende Alterung schließt, wenn im Ausgangssignal des Innensensors (1, 2) diejenigen, insbesondere schnellen Stoffkonzentrationsänderungen entsprechenden Anteile fehlen, die einem bekannten tatsächlichen zeitlichen Verhalten der Stoffkonzentration im Meßfluid entsprechen, und/oder daß die Schaltungs- und Auswerteeinheit (4) durch Einblasen vorzugsweise über die Reinigungsdüse gemäß Anspruch 15 von meßfluidfreier Druckluft in die unmittelbare Umgebung des sensitiven Bereiches des Innensensors (1, 2) eine Überprüfung insbesondere der Ansprechzeit des Innensensors (1, 2) vornimmt und das Warnsignal dann ausgibt, wenn sich der durch den Innensensor (1, 2) angezeigte Meßwert für die Stoffkonzentration nicht oder nicht mit einer erwarteten Rate verringert, und/oder daß die Schaltungs- und Auswerteeinheit (4) eine Überprüfung zumindest des Außensensors (3) durch Einleiten eines Prüfgases gestattet, wobei vorzugsweise die Schaltungs- und Auswerteeinheit (4) bei unterhalb eines vorgebbaren Grenzwertes liegenden Abweichungen des Prüfmeßwertes von dem erwarteten Meßwert eine Nachkalibrierung des Außensensors (3) veranlaßt und bei den Grenzwert überschreitenden Abweichungen das Warnsignal ausgibt.
